# EUROPÄISCHE PATENTANMELDUNG

(11) **EP 1 384 454 A1**
(43) Veröffentlichungstag der Anmeldung: **28.01.2004**
(21) Anmeldenummer: 02016768.0
(22) Anmeldetag: 26.07.2002
(51) Int. Cl.: A61F 2/38

(54) **Knieprothese**

(71) Anmelder: WALDEMAR LINK GmbH & Co. KG, 22339 Hamburg (DE)
(72) Erfinder: Keller, Arnold, 23863 Kayhude (DE)
(74) Vertreter: Glawe, Delfs, Moll & Partner

(57) **Zusammenfassung**

Knieprothese mit einem femoralen Prothesenteil (1), der ein Paar von Kondylengleitflächen (5) bildet, einem tibialen Teil (2), der mit den Kondylengleitflächen (5) zusammenwirkende Tibiagleitflächen (9) aufweist, sowie einem Kopplungsteil (10), der die femoralen und tibialen Teile (1, 2) drehbar um eine zum Schienbein etwa parallele Rotationsachse (12) miteinander verbindet. Bei gleicher AP-Ausrichtung der femoralen und tibialen Teile (1, 2) wird die Last über einen mittleren Bereich 14 der Tibiagleitflächen (9) übertragen. Erfindungsgemäß steigt die Tibiagleitfläche (9) nicht nur vor diesem Bereich (14) normalen Kontakts, sondern auch dahinter derart an, daß im Falle von Rotation der beiden Prothesenteile (1, 2) gegeneinander jede der beiden Kondylengleitflächen (5) mit der zugehörigen Tibiagleitfläche (9) vor bzw. hinter dem Bereich (14) normalen Kontakts in Berührung bleibt.

## Beschreibung

Für den Ersatz des menschlichen Kniegelenks verwendet man Prothesentypen, deren femorale und tibiale Teile je nach dem Erhaltungszustand des Bandapparats eine geringere oder stärker ausgeprägte gegenseitige Zwangsführung aufweisen. Diese bezieht sich auf die wesentlichen Bewegungsfreiheitsgrade des Knies, nämlich die Beugebewegung um eine Querachse, die Rotationsbewegung um eine etwa parallel zur Schienbeinrichtung verlaufende Rotationsachse und eine translatorische Bewegung in AP-Richtung (AP = anteroposterior). Den geringsten Grad an gegenseitiger Zwangsführung weisen sogenannte ungekoppelte Prothesen auf, die lediglich aus einem femoralen Kondylenpaar und einer tibialen Gleitfläche bestehen. Sie werden bei gut erhaltenem Bandapparat verwendet. Das andere Extrem bilden Scharnierprothesen, die bei schlecht erhaltenem Bandapparat verwendet werden und die Bewegungsmöglichkeiten des Knies auf die Beugebewegung einschränken. Zwischen diesen Extremen gibt es in unterschiedlichem Maße teilgekoppelte Systeme, die zwischen dem femoralen und dem tibialen Teil einen Zwischenteil aufweisen, der durch Bildung eines Rotationslagers die Führungsaufgaben für die Rotationsbewegung übernimmt.

Unter den teilgekoppelten Prothesen, die eine Rotationsbewegung erlauben, sind zwei Typen zu unterscheiden. Bei dem ersten Typ wird die gesamte Last über das Zwischengelenkstück übertragen, das im Verhältnis zum tibialen Teil ein Rotationslager und gegenüber dem femoralen Teil ein Beuge-Scharniergelenk bildet (DE-C-26 60 623). Da in diesem Fall die Kondylengleitflächen lediglich für eine Beugebewegung bestimmt sind, können sie kongruent zu den Gegenflächen ausgebildet sein. Die Gegenflächen sind daher konkav mit gleichem Krümmungsradius gestaltet. Der zweite Typ teilgekoppelter Prothesen überträgt die Last nicht über das Gelenkzwischenstück, sondern unmittelbar von den Kondylengleitflächen auf damit zusammenwirkende tibiale Gleitflächen (DE-A-40 38 037). In diesem Fall findet zwischen den Kondylengleitflächen und den Tibiagleitflächen nicht nur eine Beugebewegung, sondern auch die Rotationsbewegung statt. Daher dürfen die Tibiagleitflächen nicht kongruent zu den Kondylengleitflächen ausgebildet sein. Wenn sie eine freie Rotationsbewegung ermöglichen sollen, müssen die Tibiagleitflächen eben sein. In der Regel läßt man sie jedoch vor demjenigen Bereich, in welchem sie mit den Kondylenflächen bei gleicher AP-Ausrichtung des Femurteils und des Tibiateils zusammenwirken (Bereich normalen Kontakts), nach vorne hin ein wenig ansteigen. Dies hat zur Wirkung, daß bei einer Rotation diejenige Kondylengleitfläche, die sich gegenüber der Tibiagleitfläche bei der Rotation nach vorne verschiebt, angehoben wird. Dies erzeugt unter der vom Gelenk übertragenen Last ein rückdrehendes Moment, das dafür sorgt, daß die Prothesenteile, sobald dies möglich ist, wieder in ihre Normalstellung gleicher AP-Ausrichtung zurückkehren. Bei der Rotation gegenüber dem Tibiateil und der dadurch bewirkten Anhebung des Femurteils verliert die nach hinten wandernde Kondylengleitfläche ihren Kontakt mit der Tibiagleitfläche verliert. Die gesamte Last muß dann auf der anderen Seite übertragen werden, was nicht zu erhöhtem Verschleiß, sondern auch zu einem unerwünschten Biegemoment im Bereich des Rotationslagers führt.

Diesen Mangel beseitigt die Erfindung durch die Merkmale des Anspruchs 1. Demnach steigen die Tibiagleitflächen auch hinter den Bereichen des normalen Kontakts an, und zwar dergestalt, daß im Falle von Rotation jede der beiden Kondylengleitflächen mit der zugehörigen Tibiagleitfläche in Berührung bleibt; die eine Kondylengleitfläche steht in Kontakt mit dem ansteigenden Teil der Tibiagleitfläche vor dem Bereich normalen Kontakts, die andere mit dem nach hinten ansteigenden Bereich.

Vorteilhaft ist die Anwendung dieses Prinzips bei Knieprothesen mit einer gegenüber beiden Prothesenteilen fest angeordneten Rotationsachse, bei der also keine relative AP-Bewegung der Prothesenteile zueinander stattfinden kann. Der Anstieg der Tibiagleitflächen hinter dem Bereich des normalen Kontakts gleicht dann etwa demjenigen vor diesem Bereich. Die Erfindung ist aber auch anwendbar bei Prothesen, deren Rotationsachse gegenüber einem der beiden Prothesenteile in AP-Richtung verschiebbar angeordnet ist. Die Kondylengleitflächen stellen sich dann gegenüber den Tibiagleitflächen in AP-Richtung jeweils so ein, daß ausgeglichene Kraftübertragungsverhältnisse an beiden Kondylen herrschen.

Besonders einfach und übersichtlich sind die geometrischen Verhältnisse dann, wenn der Krümmungsradius des mit der Tibiagleitfläche zusammenwirkenden Teils der Kondylengleitflächen in der Beugeebene im wesentlichen konstant ist, d.h. wenn die Kondylengleitflächen kreisbogenförmig gestaltet sind. Die Erfindung ist aber auch anwendbar, wenn dies nicht der Fall ist. Wenn der Verlauf der Kondylenflächen unregelmäßig ist, ist es dabei allerdings zweckmäßig, eine relative Bewegungsmöglichkeit der femoralen und tibialen Teile in AP-Richtung vorzusehen. Dies ist nicht notwendig, wenn die Kondylengleitflächen nach einer archimedischen Spirale geformt sind. Das Profil der Kondylengleitflächen sollte im allgemeinen konstant sein

Die Erfindung wird im folgenden näher unter Bezugnahme auf die Zeichnung erläutert, die ein vorteilhaftes Ausführungsbeispiel veranschaulicht. Es zeigen:
- Fig. 1: eine Seitenansicht der Prothese,
- Fig. 2: eine Draufsicht auf die tibialen Gleitflächen,
- Fig. 3: einen Schnitt durch die tibialen Gleitflächen und
- Fig. 4 und 5: zwei Seitenansichten von entgegengesetzten Seiten bei rotiertem Femurteil.

Die Prothese weist einen Femurteil 1 und einen Tibiateil 2 auf, die über Dornen 3, 4 in bekannter Weise am unteren Ende des Oberschenkelknochens (Femur) bzw. am oberen Ende des Schienbeins (Tibia) zu verankern sind. Der Femurteil 1 weist ein Paar von Kondylengleitflächen 5 auf, die sich an der Vorderseite zu einer Patellagleitfläche 6 vereinigen. Der Tibiateil 2 bildet oben eine Tragplatte 7, auf der das sogenannte Tibiaplateau 8 aus einem gleitgünstigen Werkstoff, beispielsweise Polyethylen, verankert ist, das die Tibiagleitflächen 9 bildet, auf denen die vorzugsweise von poliertem Metall gebildeten Kondylengleitflächen 5 gleiten. Der Femurteil 1 und der Tibiateil 2 sind miteinander durch einen Zwischenteil 10 gekoppelt, der einerseits mit dem Femurteil 1 ein Beugelager mit der Achse 11 und andererseits mit dem Tibiateil ein Rotationslager mit der Achse 12 bildet. Einzelheiten dieser Konstruktion sind erläutert in den europäischen Patentanmeldungen 1 110 261 und 1 111 551, auf die hiermit Bezug genommen wird und deren Offenbarungsinhalt zum Gegenstand der vorliegenden Anmeldung gemacht wird.

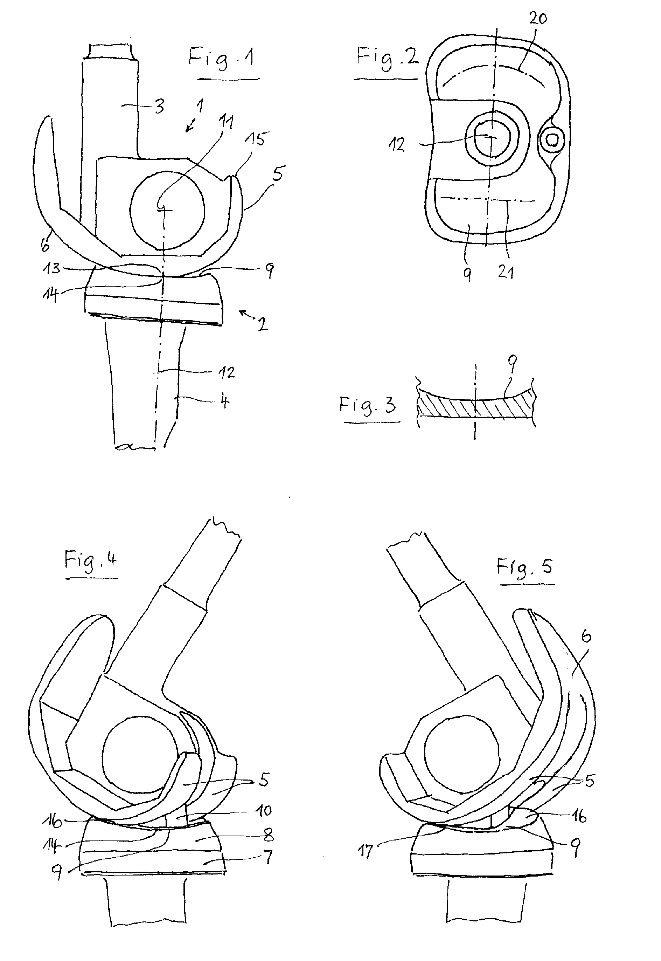

In unrotierter Stellung (Fig. 1) ruhen die femoralen Gleitflächen 5 mit ihrem Bereich 13, dessen Richtung etwa lotrecht zum Radius verläuft, auf dem Bereich 14 normalen Kontakt der tibialen Gleitflächen 9, dessen Richtung etwa lotrecht zur Achse 12 verläuft. Bei Beugebewegung kann der zwischen dem Bereich 13 und dem hinteren Ende 15 der femoralen Gleitflächen liegende Abschnitt tragend mit der Tibiagleitfläche 9 in Berührung kommen. Dieser Abschnitt verläuft im dargestellten Beispiel kreisbogenförmig mit konstantem Radius zur Beugeachse 11. Das Gleitflächenprofil ist in diesem Abschnitt konstant.

Der Bereich 14 der Tibiagleitfläche 9 hat dasselbe Profil (im Frontalschnitt) wie der damit zusammenwirkende Abschnitt 13-15 der zugehörigen Femurgleitfläche. Das bedeutet, daß im unrotierten Zustand theoretischer Linienkontakt herrscht. Praktisch ergibt sich Flächenkontakt infolge der Nachgiebigkeit des Werkstoffs der Tibiagleitflächen 9.

Der vor dem Bereich 13 liegende Abschnitt 6 der Kondylengleitflächen und der Patellagleitfläche ist für die Übertragung der Lastkräfte auf den Tibiateil 2 der Prothese ohne Belang.

Die Tibiagleitfläche 9 ist in der Sagittalebene schwach konkav gekrümmt, wie es Fig. 3 zeigt. Der Krümmungsradius ist beträchtlich größer als der Radius des femoralen Gleitflächenabschnitts 13-15. Dies ist erforderlich, damit die femoralen Gleitflächen im Falle von Rotation sich - ausgehend von dem Bereich normalen Kontakts 14 - im wesentlichen ungehindert um eine geringe Distanz vor- und zurückbewegen können. Im Falle einer kräftigen Rotation verlassen die Kondylengleitflächen 5 den Bereich 14 normalen Kontakts. Auf der einen Seite (siehe Fig. 4) bewegen sie sich in den ansteigenden Abschnitt 16 der Tibiagleitflächen, der vor dem Bereich normalen Kontakts 14 liegt. Auf der anderen Seite (Fig. 5) bewegen sie sich im hinteren, ansteigenden Abschnitt 17 der Tibiagleitflächen 9.

Die Tibiagleitflächen sind so geformt, daß die Kondylengleitflächen 5 im Falle einer solchen Rotation auf beiden Seiten Kontakt mit der Tibiagleitfläche 9 behalten, nämlich auf der einen Seite mit dem vorderen Abschnitt 16 und auf der anderen Seite mit dem hinteren Abschnitt 17.

Will man in diesen Abschnitten Linienkontakt zwischen den Kondylengleitflächen 5 und den Tibiagleitflächen 9 aufrecht erhalten, so muß man die Tibiagleitflächen 9 so formen, daß sie in Richtung von Kreisbögen 20 um die Rotationsachse 12 in einer Schnittebene, die diese Rotationsachse enthält, dasselbe Profil wie die Kondylengleitflächen 5 aufweisen. Dies läßt sich ohne weiteres mit Hilfe eines Werkzeugs bewerkstelligen, das das Profil der Kondylengleitflächen aufweist und um die Achse 12 rotiert wird. Dies ist jedoch verhältnismäßig aufwendig. Einfacher ist es, die Tibiagleitflächen 9 mittels Werkzeugen zu fräsen, die in AP-Richtung 20 bewegt werden. In diesem Fall verzichtet man bei Rotation auf den idealen Linienkontakt zwischen den Kondylengleitflächen 5 und den Tibiagleitflächen 9 um so stärker, je weiter sich der Punkt des jeweiligen Kontakts von dem Bereich 14 normalen Kontakts entfernt. Dies ist aber unschädlich, weil so starke Rotation vergleichsweise selten stattfindet und die Perioden lang dauernder Lastübertragung auf den Bereich 14 normalen Kontakts beschränkt sind. Entscheidend ist, daß bei einer solchen starken Rotation nicht nur eine der beiden Kondylengleitflächen mit der Tibiagleitfläche zusammenwirkt, sondern beide.

## Patentansprüche

1. Knieprothese mit einem femoralen Prothesenteil (1), der ein Paar von Kondylengleitflächen (5) bildet, einem tibialen Teil (2), der mit den Kondylengleitflächen (5) zusammenwirkende Tibiagleitflächen (9) aufweist, sowie einem Kupplungsteil (10), der die femoralen und tibialen Teile (1, 2) drehbar um eine zum Schienbein etwa parallele Rotationsachse (12) miteinander verbindet, wobei die Tibiagleitflächen einen Bereich (14) normalen Kontakts aufweisen, welcher mit der zugehörigen Kondylengleitfläche (5) bei gleicher AP-Ausrichtung der femoralen und tibialen Teile (1, 2) zusammenwirkt und vor dem Bereich (14) normalen Kontakts mit einem Krümmungsradius ansteigen, der wesentlich größer als der Krümmungsradius des mit der Tibialgleitfläche zusammenwirkenden Teils (13-15) der Kondylengleitfläche (5) ist, **dadurch gekennzeichnet, daß** die Tibiagleitflächen (9) auch hinter dem Bereich (14) normalen Kontakts dergestalt ansteigen, daß im Falle von Rotation jede der beiden Kondylengleitflächen (5) mit der zugehörigen Tibialgleitfläche (9) vor bzw. hinter dem Bereich (14) normalen Kontakts in Berührung bleibt.

2. Prothese nach Anspruch 1, **dadurch gekennzeichnet, daß** die Rotationsachse (12) gegenüber beiden Prothesenteilen (1, 2) fest angeordnet ist.

3. Prothese nach Anspruch 1, **dadurch gekennzeichnet, daß** die Rotationsachse (12) gegenüber einem der beiden Prothesenteile (1, 2) in AP-Richtung verschiebbar angeordnet ist.

4. Prothese nach einem der Ansprüche 1 bis 3, **dadurch gekennzeichnet, daß** der Krümmungsradius des mit der Tibiagleitfläche (9) zusammenwirkenden Teils (13-15) der Kondylengleitfläche (5) in der Beugeebene im wesentlichen konstant ist.
